# FASCICULE DE BREVET EUROPEEN

(11) **EP 2 025 544 B1**
(45) Date de publication et mention de la délivrance du brevet: **24.02.2010**
(21) Numéro de dépôt: 08159493.9
(22) Date de dépôt: 02.07.2008
(51) Int. Cl.: B60H 3/00

(54) **Dispositif de diffusion d'agents volatils muni d'un moyen d'extraction de cartouche**
Diffusionsvorrichtung für flüchtige Wirkstoffe, ausgestattet mit einem Mittel zum Entfernen von Patronen
Device for diffusing volatile agents, provided with a cartridge extraction means

(30) Priorité: 02.07.2007 FR 0704768
(43) Date de publication de la demande: 18.02.2009
(73) Titulaire: Valeo Systèmes Thermiques, 78321 Le Mesnil St Denis Cedex (FR)
(72) Inventeur: Feuillard, Vincent Valeo Systemes Thermiques, 78320 Le Mesnil Saint Denis (FR); Elliot, Gilles Valeo Systemes Thermiques, 91080 Courcouronnes (FR); Loup, Didier Valeo Systemes Thermiques, 78310 Maurepas (FR); Stanek, Pietr Valeo Systemes Thermiques, 50801 Horice (CZ)
(74) Mandataire: Léveillé, Christophe

(56) Documents cités:
- EP-A- 1 561 618
- DE-U1-202004 010 015
- FR-A- 2 815 294

## Description

La présente invention concerne un dispositif de diffusion d'agents volatils dans un véhicule automobile.

L'invention trouve une application particulièrement avantageuse dans le domaine de la diffusion de fragrances à l'intérieur des véhicules automobiles.

Dans le domaine de la diffusion d'agent volatil dans un habitacle de véhicule, il existe plusieurs types de diffuseur. Par exemple, on connaît un dispositif de diffusion logé dans le système de ventilation, chauffage et/ou climatisation du véhicule.

Cependant, ce dispositif de diffusion d'agents volatils connu de l'état de la technique présente l'inconvénient d'exiger de la part de l'automobiliste utilisateur qu'il réalise une opération manuelle pour remplacer une cartouche usée par une cartouche neuve. En effet, le système de ventilation, chauffage et/ou climatisation se trouvant généralement sous la planche de bord du véhicule, on comprend que le remplacement de la cartouche nécessite le démontage de plusieurs éléments du véhicule, tels que la planche de bord elle-même et la console centrale. En conséquence, un utilisateur ne peut pas changer facilement la cartouche d'agent volatil.

On connaît également un dispositif de diffusion comprenant un boîtier comportant une entrée d'air ambiant, une sortie d'air traité et une chambre de diffusion dans laquelle est placé une cartouche contenant un agent volatil. Ce dispositif de diffusion est intégré dans une planche de bord entre deux aérateurs. L'entrée d'air ambiant et la sortie d'air traité se situent dans un des aérateurs. Ce type de dispositif présente l'inconvénient d'être manuel. En d'autres termes, le changement de l'agent volatil nécessite une manipulation importante du passager consistant à déverrouiller la cartouche contenant l'agent volatil, retirer la mèche de la cartouche, introduire une mèche neuve dans la cartouche et enfin reloger la cartouche dans le boîtier.

EP 1 561 618 divulgue un dispositif selon le préambule de la revendication 1.

Aussi, un but de l'invention est de proposer un dispositif de diffusion d'agents volatils qui donnerait à l'utilisateur la possibilité de remplacer une cartouche par une autre de manière très simple, quasi automatique, sans avoir à effectuer de manipulations complexes et fastidieuses.

Ce but est atteint, conformément à l'invention, grâce à un dispositif de diffusion d'au moins un agent volatil dans un véhicule automobile, comprenant un boîtier comportant une entrée d'air ambiant, une sortie d'air traité et une chambre de diffusion apte à recevoir un insert, au moins une cartouche d'agent volatil, ledit dispositif comprenant en outre un actionneur entraînant l'insert en rotation autour de l'axe A, remarquable en ce que ledit insert comprend un berceau portant ladite cartouche, ledit berceau étant lié en rotation à l'insert et monté libre en translation axiale sur l'insert, et en ce que ledit dispositif comprend des moyens d'extraction dudit berceau hors dudit boîtier par translation axiale parallèlement à l'axe A.

Ainsi, en agissant sur lesdits moyens d'extraction, il est possible de dégager le berceau hors du boîtier et donc d'accéder à la ou les cartouches qui s'y trouvent, ce qui permet d'effectuer un remplacement de cartouche de manière très simple.

On notera également que l'invention présente l'avantage de pouvoir placer plusieurs cartouches dans le berceau, et non une seule comme dans le dispositif connu. L'automobiliste peut alors choisir celle des cartouches qu'il souhaite utiliser.

Selon un mode de réalisation de l'invention, lesdits moyens d'extraction comprennent un tiroir d'extraction logé dans la chambre de diffusion et contenant ledit insert, et des moyens d'entraînement en translation axiale dudit tiroir hors du boîtier, le tiroir d'extraction étant apte à entraîner ledit berceau hors du boîtier sous l'action desdits moyens d'entraînement en translation axiale.

De manière à éviter qu'une cartouche puisse être remplacée alors que, par exemple, le dispositif de diffusion est en marche, l'invention prévoit que lesdits moyens d'entraînement sont bloqués pour toute position angulaire du berceau autour de l'axe A différente d'une position angulaire d'extraction donnée. En d'autres termes, il est nécessaire pour remplacer une cartouche d'amener le berceau, par rotation autour de l'axe A, dans une position angulaire spécifique, dites d'extraction, les moyens d'entraînement par translation axiale du tiroir ne pouvant être mis en oeuvre que lorsque le berceau se trouve dans une position angulaire d'extraction.

Avantageusement, lesdits moyens d'entraînement en translation axiale sont activés par une action de pression axiale sur ledit tiroir d'extraction. Ce mode d'activation est particulièrement simple et ne demande que très peu d'effort et d'attention.

Plus précisément, ledit dispositif comprend des moyens de verrouillage du tiroir d'extraction dans le boîtier, lesdits moyens de verrouillage étant aptes à être libérés sous ladite action de pression axiale. Les moyens de verrouillage du tiroir étant ainsi libérés par la pression axiale exercée par l'utilisateur, les moyens d'entraînement peuvent alors extraire le tiroir hors du boîtier.

Afin de rendre effective la disposition précédente interdisant tout remplacement de cartouche hors positions d'extraction du berceau, il est prévu par l'invention que le tiroir d'extraction et l'insert comportent des moyens de butée destinés à bloquer la libération des moyens de verrouillage sous ladite action de pression axiale lorsque le berceau est dans une position angulaire différente de ladite position angulaire d'extraction.

Selon une forme de réalisation particulière de l'invention, ladite chambre de diffusion présente un secteur de plus grand diamètre destiné à limiter la compression radiale de moyens d'étanchéité de la cartouche en position d'extraction. Cette caractéristique permet de faciliter le mouvement de translation du tiroir en réduisant les frottements introduits par lesdits moyens d'étanchéité.

Afin d'empêcher le dispositif de diffusion de fonctionner alors que le tiroir est en position ouverte, l'invention prévoit d'équiper ledit dispositif d'un détecteur d'ouverture du tiroir apte à fournir un signal de mise hors fonctionnement dudit dispositif lorsque le tiroir est hors du boîtier.

Selon l'invention, le tiroir est muni d'un bouchon comportant des moyens de distribution radiale de l'air traité. En particulier, ledit bouchon est un cylindre creux d'axe A, lesdits moyens de distribution radiale étant constitués par au moins un trou latéral ménagé dans ledit cylindre.

Ce système de distribution de l'agent volatil présente le double avantage d'être plus esthétique et plus efficace que les systèmes connus en termes d'homogénéité de l'air traité à l'intérieur du véhicule.

La description qui va suivre en regard des dessins annexés, donnés à titre d'exemples non limitatifs, fera bien comprendre en quoi consiste l'invention et comment elle peut être réalisée.
La figure 1 est une vue en perspective éclatée d'un dispositif de diffusion conforme à l'invention.
La figure 2 est une vue en perspective du berceau du dispositif de la figure 1 en position OFF.
La figure 3 est une vue de face du berceau du dispositif de la figure 1 en position ON Maxi pour une cartouche.
La figure 4 est une vue de face du berceau du dispositif de la figure 1 en position de remplacement d'une cartouche.
La figure 5 est un diagramme des positions angulaires de l'actionneur du dispositif de la figure 1
La figure 6 est une vue en perspective de moyens de verrouillage du tiroir d'extraction dans le boîtier du dispositif de la figure 1.

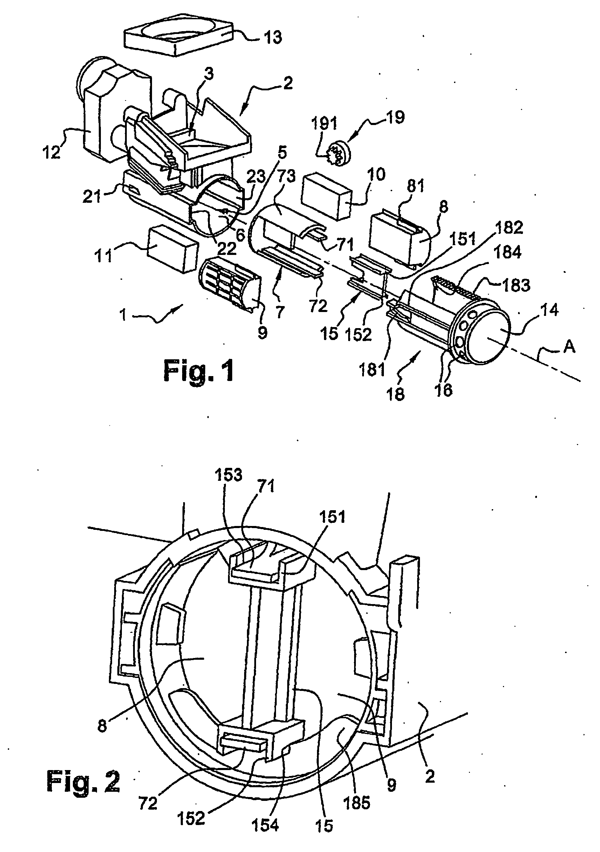

Sur la figure 1 est représenté en vue éclatée un dispositif 1 de diffusion d'agents volatils dans un véhicule automobile. Ces agents volatils sont généralement des fragrances imprégnées dans des mèches qui, dans l'exemple de la figure 1, sont au nombre de deux, respectivement référencées 10 et 11, correspondant à des fragrances différentes. En pratique, les mèches 10, 11 sont insérées dans des cartouches 8, 9 de façon à être montées dans le dispositif 1 de diffusion d'une manière qui sera expliquée plus loin.

Le dispositif 1 de diffusion comprend un boîtier 2 comportant une entrée 3 d'air ambiant provenant d'un ventilateur 13 et destiné à pénétrer dans une chambre 6 de diffusion dans laquelle sont disposées les cartouches 8, 9 d'agents volatils. Comme le montre la figure 1, la chambre 6 de diffusion affecte une forme sensiblement cylindrique d'axe A, lequel constitue l'axe autour duquel s'organisent les fonctions principales du dispositif 1 de diffusion.

Au passage dans la chambre 6 de diffusion, l'air ambiant se charge d'une ou de l'autre des fragrances. L'air ainsi parfumé sort de la chambre 6 de diffusion par une sortie 5 d'air traité en direction d'un bouchon 14 comportant des moyens de distribution radiale autour de l'axe A de l'air traité issu de la sortie 5. Dans l'exemple de réalisation de la figure 1, ledit bouchon 14 se présente sous la forme d'un cylindre creux d'axe A, lesdits moyens de distribution radiale sont alors des trous latéraux 16 ménagés dans la paroi cylindrique du bouchon 14.

Les avantages de ce mode de distribution de l'air traité sont multiples. Du point de vue esthétique, le bouchon 14 permet de remplacer une ouverture et une grille en façade généralement disgracieuses par un élément où les trous de distribution sont beaucoup plus discrets et donc moins visibles. D'un point de vue technique, la sortie latérale à travers les trous 16 réoriente l'air traité en direction des aérateurs, entre lesquels est installé le dispositif 1 de diffusion. De cette façon, on obtient rapidement un mélange homogène de l'air traité avec l'air issu des aérateurs, ainsi qu'une diffusion et une perception rapides de l'air parfumé partout dans le véhicule.

On peut voir sur la figure 1 que les cartouches 8, 9 sont montées sur un berceau 15, lequel est introduit dans un insert cylindrique 7 coaxial à la chambre 6 de diffusion par coulissement de poutres 71, 72 de l'insert 7 dans des rails 151, 152 du berceau 15. Lorsque l'insert 7 muni du berceau 15 portant les cartouches 8, 9 est placé à l'intérieur de la chambre 6 de diffusion, un actionneur 12, un moteur pas à pas par exemple, permet d'entraîner en rotation autour de l'axe A l'insert 7 et donc le berceau 15 et les cartouches 8, 9 puisque, par construction, le berceau 15 est lié en rotation à l'insert 7. Par contre, le berceau 15 reste monté libre en translation axiale, le long de l'axe A, sur l'insert 7.

En faisant tourner l'insert 7 autour de l'axe A, l'actionneur 12 permet d'amener les cartouches dans les différentes positions fonctionnelles suivantes.

Dans la position de la figure 1, qui correspond également à celle de la figure 2, le dispositif 1 de diffusion est hors fonctionnement (position dite OFF) car, dans ce cas, une paroi 73 de l'insert 7 occulte totalement l'entrée 3 d'air ambiant.

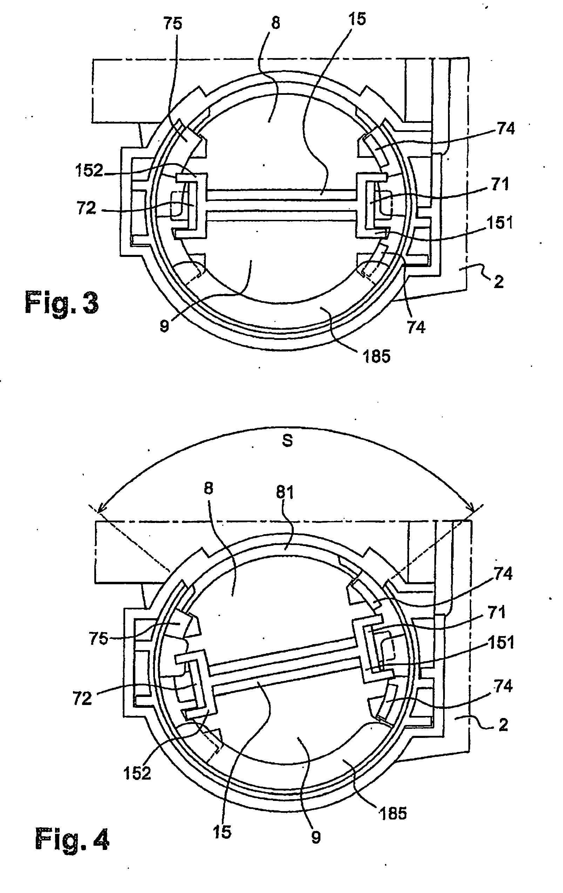

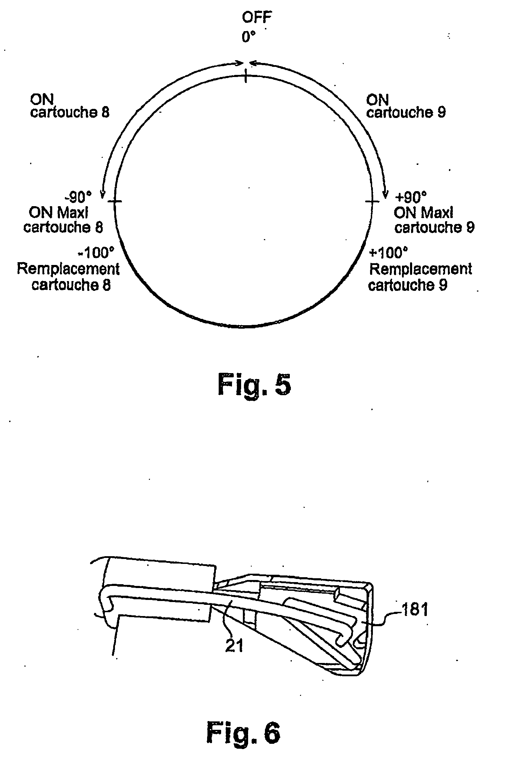

Lorsque l'actionneur 12 fait tourner l'insert 7 de 90° dans un sens ou dans l'autre à partir de la position OFF, la cartouche 8 ou la cartouche 9 est amenée dans une position de diffusion maximale (position ON Maxi) comme indiqué sur la figure 3 où la cartouche 8 est dans la position ON Maxi. Des positions ON intermédiaires entre OFF et ON Maxi sont bien entendu possibles. On a représenté sur la figure 5 la position OFF de l'actionneur 12 ainsi que les positions angulaires ON Maxi à ±90° de la position OFF. Dans une position ON, l'air ambiant introduit à travers l'entrée 3 se charge de la fragrance correspondant à la cartouche active, la cartouche 8 dans l'exemple illustré sur la figure 3, et circule dans le dispositif 1 jusqu'au bouchon 14 de distribution radiale.

De manière à permettre à l'utilisateur de changer une cartouche usée par une cartouche neuve, le dispositif 1 de diffusion de la figure 1 comprend des moyens d'extraction automatique du berceau 15 hors du boîtier qui vont maintenant être décrits en détail.

Comme l'indique la figure 1, ces moyens d'extraction comprennent un tiroir 18 d'extraction de forme sensiblement cylindrique d'axe A qui, en mode de diffusion du dispositif 1, est logé dans la chambre 6 de diffusion et contient l'insert 7 avec le berceau 15. Le tiroir 18 n'est pas couplé à l'actionneur 12 de sorte qu'il ne subit aucun mouvement de rotation, même lorsque l'actionneur 12 fonctionne, dans ce cas, seuls l'insert 7 et le berceau 15 peuvent tourner autour de l'axe A.

Lesdits moyens d'extraction comprennent en outre des moyens d'entraînement en translation axiale, le long de l'axe A, du tiroir 18 hors du boîtier 2. D'autre part, il est prévu que dans son mouvement de translation axiale le tiroir 18 entraîne également hors du boîtier 2 le berceau 15 dont on rappelle qu'il est libre en translation par rapport à l'insert 7. On comprend alors que, si la translation axiale est effectuée sur une distance au moins égale à la longueur L des cartouches 8, 9 prise parallèlement à l'axe A, il soit possible de dégager complètement une des cartouches et ainsi de la remplacer si elle est usée.

Dans un mode de réalisation avantageux, les moyens d'entraînement en translation axiale du tiroir 18 sont choisis du type dit « push-pull », c'est-à-dire activés par une action de pression axiale de l'utilisateur sur le tiroir 18 d'extraction.

Plus précisément, comme l'indique la figure 1, ces moyens sont constitués par des ressorts de compression, non représentés, disposés dans des gorges 22, 23 ménagées dans la chambre 6 de diffusion et coopérant avec des guides 182 du tiroir 18. Lorsque des moyens de verrouillage du tiroir 18 dans le boîtier sont libérés par pression axiale, les ressorts initialement comprimés peuvent alors se détendre et appliquer au tiroir un mouvement de translation axiale propre à extraire le tiroir 18 et le berceau 15 hors du boîtier 2.

Sur les figures 1 et 6, on a représenté des moyens de verrouillage du tiroir 18 dans le boîtier 2 constitués par une came 181 ménagée sur le tiroir 18 coopérant avec un ressort 21 de flexion disposé sur le boîtier. En mode de diffusion du dispositif 1, le ressort 21 de flexion retient le tiroir 18 dans le boîtier. Si l'utilisateur décide de changer l'une des cartouches 8, 9, il exerce une pression sur le bouchon 14 sur une course de quelques millimètres qui a pour effet de libérer la came 181 du ressort 21 de flexion et donc de permettre aux ressorts de compression d'exercer leur fonction d'entraînement axial du tiroir 18 hors du boîtier 2.

Dans le but de maîtriser la vitesse d'extraction du tiroir 18 et éviter des claquements en fin de course, des moyens d'amortissement du mouvement de translation du tiroir 18 sont prévus, constitués par exemple d'une crémaillère 183 disposée longitudinalement sur le tiroir 18 et un amortisseur 19 à graisse comportant un pignon 191 coopérant avec la crémaillère 183.

Après avoir changé de cartouche, l'utilisateur referme le tiroir 18 en le poussant manuellement contre l'effort des ressorts de compression jusqu'à ce que le ressort 21 de flexion verrouille la came 181.

Afin d'empêcher tout remplacement de cartouche alors que le dispositif 1 de diffusion est en position OFF ou dans une position ON, l'invention prévoit qu'un tel remplacement n'est possible que si le berceau 15 est amené en rotation autour de l'axe A par l'actionneur 12 dans l'une ou l'autre de deux positions angulaires d'extraction spécifiques correspondant chacune au remplacement de l'une des cartouches 8 ou 9, ainsi que le montre la figure 5. Ces positions d'extraction s'écartent d'environ 10° par rapport aux positions ±90° ON Maxi. La course angulaire totale de l'actionneur est donc de 200° environ. La figure 4 montre plus particulièrement la cartouche 8 dans sa position angulaire d'extraction.

Lorsque le berceau 15 est dans une position angulaire d'extraction correspondant au remplacement de l'une ou l'autre des cartouches 8, 9, le tiroir 18 peut entraîner le berceau 15 hors du boîtier 2 grâce à un ergot 184 d'accrochage disposé à l'extrémité distale du tiroir et coopérant avec le berceau 15.

Hors de ces positions angulaires d'extraction spécifiques, les moyens de verrouillage du tiroir 18 dans le boîtier 2 sont bloqués en position de verrouillage et donc insensibles à toute action de pression axiale sur le tiroir 18. C'est ce qui est représenté sur les figures 2 et 3, où le berceau 15 est montré dans des positions angulaires OFF et ON maxi, donc différentes des positions angulaires d'extraction. On voit sur ces figures des moyens de butée destinés à limiter la course du tiroir 18 sous l'action d'une pression axiale à une valeur inférieure, voire nulle, à la course de quelques millimètres nécessaire pour libérer la came 181.

Ces moyens de butée, représentés sur les figures 2 et 3, comprennent un mur 185 ménagé sur un secteur périphérique du tiroir 18.

Dans le cas de la figure 2, position OFF, des encoches 153, 154 portées par le berceau 15 viennent enserrer le mur 185 de sorte que le tiroir 18 se trouve immobilisé en translation axiale et donc dans l'impossibilité d'effectuer la course nécessaire à la libération des moyens de verrouillage. Toute action de pression axiale est donc neutralisée.

Dans le cas de la figure 3, une butée proprement dite 74 est disposée sur l'insert 7 de sorte que, dans toutes les positions différentes des positions angulaires d'extraction, ON Maxi dans l'exemple de la figure 3, ladite butée 74 soit en contact avec le mur 185 ou du moins à une distance inférieure à la course de libération des moyens de verrouillage. Là encore toute action de pression axiale sur le tiroir 18 est neutralisée car le mur 185 est bloqué contre la butée 74.

Par contre, dans le cas de la figure 4 où le berceau est dans une position angulaire d'extraction, la butée 74 est décalée par rapport au mur 185 permettant ainsi au tiroir 18 d'effectuer la course de libération de la came 181. Une deuxième butée 75, décalée en profondeur par rapport à la première butée 74, permet au besoin de limiter cette course à la valeur minimale requise de quelques millimètres afin d'éviter les effets d'une trop forte action de pression sur le tiroir 18.

Enfin, on peut voir sur la figure 4 que le diamètre de la chambre 6 de compression est plus grand sur un secteur S où une lèvre 81 d'étanchéité de la cartouche 8 en position de remplacement est en contact avec la paroi intérieure de la chambre 6. Cette disposition présente l'avantage de limiter les frottements parasites dus à la compression de cette lèvre d'étanchéité contre la paroi de la chambre 6 lors de l'extraction du tiroir 18. Le mouvement de translation du tiroir 18 est donc facilité.

Bien entendu, le choix de la cartouche active, le réglage d'intensité de la diffusion (positions ON), l'arrêt de la diffusion (position OFF) et la demande de remplacement d'une cartouche donnée peuvent être effectués par l'utilisateur au moyen d'un tableau électronique de commande situé sur la planche de bord du véhicule.

Afin d'éviter que le dispositif 1 de diffusion puisse être placé dans une position ON alors que le tiroir 18 se trouve à l'extérieur du boîtier 2 lors d'un remplacement de cartouche, un détecteur d'ouverture du tiroir 18, non représenté, est placé sur le boîtier 2 et fournit au tableau de commande un signal de mise hors fonctionnement du dispositif 1.

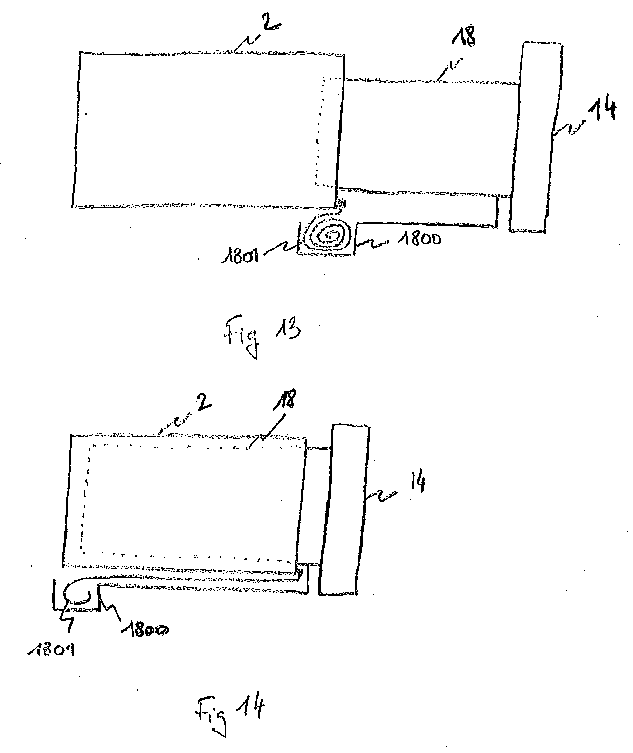

Selon un autre mode de réalisation représenté en figures 13 et 14, les moyens d'entraînement sont des ressorts dits « à force constante ». En effet, les ressorts de compression précédemment utilisés présentent certains inconvénients. L'effort des ressorts à compression n'est pas constant sur la course du tiroir 18. Ceci implique les effets néfastes suivants :
- quand le tiroir est fermé : un effort important est exercée sur la came de verrouillage.
- quand le tiroir s'ouvre : l'effort du ressort décroît rapidement, ce qui se traduit par une ouverture brutale au début puis lente en fin de course. L'utilisateur perçoit alors le dispositif comme fragile et de pauvre qualité.
- quand le tiroir est ouvert : un effort nul impliquant un risque de finir l'ouverture du tiroir à la main, faute de pression des ressorts (pression faible annulée par les frottements).

Les ressorts dits « à force constante » présentent les avantages suivants :
- effort constant sur toute la course du tiroir 18 (effort dû à la torsion d'une spire se déroulant). Cet effort constant est perçu par l'utilisateur comme un gage de qualité du dispositif.
- quand le tiroir est fermé : effort de verrouillage/déverrouillage faible (effort réduit sur la came de verrouillage)
- quand le tiroir s'ouvre : la vitesse d'ouverture est constante sur toute la course (sans assistance d'un moteur)
- quand le tiroir est ouvert : pas de risque de finir l'ouverture à la main

Selon ce mode de réalisation représenté en figure 13 et 14, le tiroir 18 comporte à son bord un logement 1800 dans lequel est installé un ressort 1801 dit « à force constante ». Ce ressort 1801 est une bande de métal enroulée sur elle-même selon une position de repos. Une extrémité du ressort 1801 est fixée au boîtier 2 et une autre extrémité est fixée au logement 1800.

En figure 13, le tiroir 18 est ouvert. Dans cette configuration, une cartouche est extractible du tiroir 18. Le ressort 1800 est quant à lui dans une position de repos dans laquelle il est complètement enroulé sur lui-même.

En figure 14, le tiroir 18 est fermé, les cartouches 7, 8 se trouvant à l'intérieur de la chambre 6. Le ressort 1800 est alors dans une position de déroulement total dans laquelle est il déroulé. Il exerce alors une force qui tend à ouvrir le tiroir 18. Les moyens de verrouillage précédemment décrits empêchent l'ouverture du tiroir 18 sans une intervention manuelle.

Selon encore un autre mode de réalisation, le dispositif de diffusion 1 ne nécessite plus aucune action manuelle de l'utilisateur pour ouvrir ou fermer le tiroir 18 afin de extraire une cartouche 8, 9 ne contenant plus d'agent volatil. En effet, selon ce mode de réalisation, l'actionneur 12 contrôle à la fois le mouvement de rotation de l'insert 7 et le mouvement de translation du tiroir 18.

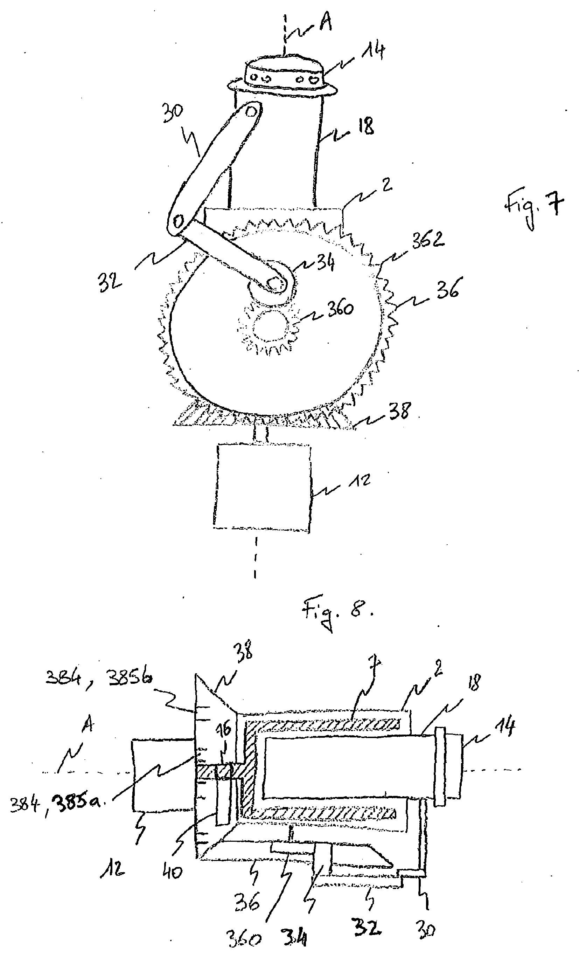

Ici, le dispositif de diffusion 1 comprend le boîtier 2, l'insert 7, le berceau 15 le tiroir 18 et le bouchon 14. Comme illustré à la figure 7, le tiroir 18 est relié à une première bielle 30, laquelle est elle-même reliée à une deuxième bielle 32. L'extrémité de la deuxième bielle 32 non reliée à la première bielle 30 comporte un pignon 34 coopérant avec une première roue dentée 36. Cette première roue dentée 36 présente un premier anneau denté 360 agissant avec le pignon 34 et un deuxième anneau denté 362 coopérant avec une deuxième roue dentée 38 mise en rotation par l'actionneur 12. De la sorte, lorsque l'actionneur 12 est en fonctionnement, il entraîne en rotation la deuxième roue dentée 38, laquelle entraîne en rotation la première roue dentée 36. La rotation de la première roue dentée 36 provoque la rotation de la deuxième bielle 32, laquelle entraîne en mouvement la première bielle 30. En conséquence, le tiroir 18 est extrait en translation du boîtier 2 sous l'action de la première bielle 30, le tiroir 18 entraînant avec lui le berceau 15 comme expliqué précédemment. L'ensemble formé par la première bielle 30, la deuxième bielle 32, la première roue dentée 36 et la deuxième roue dentée 38 est alors le moyen d'extraction du tiroir 18 du boîtier 2 pour le remplacement d'une cartouche 8, 9. Ce moyen d'extraction est commandé par l'actionneur 12.

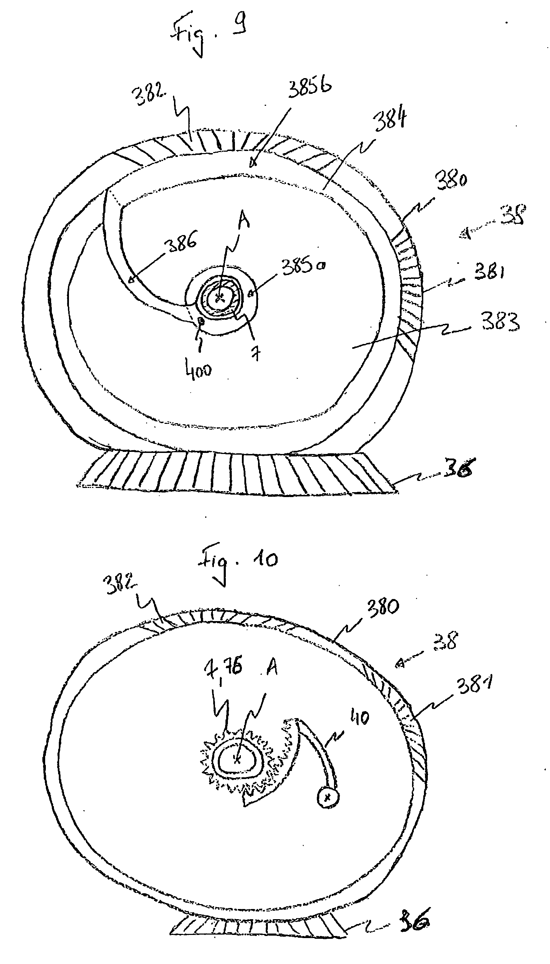

En figure 9 est représenté plus en détail la deuxième roue dentée 38. Cette deuxième roue dentée 38 comprend un collier 380 circulaire dont deux portions sont munies de dents. De la sorte, la deuxième roue dentée 38 comprend une première portion dentée 381 et une deuxième portion dentée 382, les deux portions 381, 382 étant séparées l'une de l'autre. Lorsque la deuxième roue dentée 38 tourne autour de l'axe A, elle entraîne en rotation la première roue dentée 36 par l'intermédiaire soit de la première portion dentée 381 soit de la deuxième portion dentée 382. En effet, entre les deux portions dentées 381, 382, le collier 380 n'est pas muni de dent et ne peut coopérer mécaniquement avec la première roue dentée 36.

Cependant, lorsque la deuxième roue 38 tourne sans entraîner en rotation la première roue dentée 36, elle permet d'actionner le mouvement de rotation de l'insert 7 à l'intérieur du boîtier 2.

A cet égard, le dispositif de diffusion selon ce mode de réalisation est représenté en figure 8 selon une vue en coupe comme vu précédemment, l'actionneur 12 entraîne la deuxième roue dentée 38 lorsqu'un utilisateur commande le dispositif de diffusion via une interface de commande non représentée et placée au niveau du tableau de bord du véhicule. Cette deuxième roue dentée coopère avec la première roue dentée 36 pour ouvrir ou fermer le dispositif de diffusion 1, c'est-à-dire extraire ou insérer le tiroir 18 du boîtier 2. Le mouvement de translation du tiroir 18 est assuré par la première 30 et la deuxième 32 bielles, lesquelles sont mises en mouvement par la première roue dentée 36 via le pignon 34. L'insert 7 comporte dans ce mode de réalisation une roue crantée 76 coopérant avec une crémaillère 40. Cette dernière 40 est en interaction avec un chemin de came 384 formé sur la deuxième roue dentée 38. Le mécanisme pour le pivotement de l'insert 7 va être explicité maintenant.

En figures 9 et 10 est représenté la première roue dentée 36 et la deuxième roue dentée 38. Cette dernière 38 comprend une surface radiale 383 sur laquelle un chemin de came 384 est formé. Constitué d'une première 385a et d'une deuxième 385b courses mortes et d'une course 386 pour le pivotement de l'insert 7, ce chemin de came 384 coopère avec un pointeur 400 pour entraîner en rotation la crémaillère 40, le pointeur 400 étant formé sur la crémaillère 40. On entend par « course pour le pivotement de l'insert » la portion de chemin de came 384 dans laquelle le pointeur 400 se déplace et entraîne en rotation la crémaillère 40. La roue crantée 76, formée sur l'insert 7, coopère avec la crémaillère 40 pour faire tourner l'insert 7. Une explication plus détaillée de l'engrenage constitué de la deuxième roue dentée 38, de la crémaillère 40 et de la roue crantée 76 va être donnée ci-dessous.

La deuxième roue dentée 38 munie du chemin de came 384 coopère avec le pointeur 400 lorsque le tiroir 18 est à l'intérieur du boîtier 2. Selon cette figure 9, le pointeur 400 est situé dans la première course morte 385a. On entend par « course morte » une portion du chemin de came 384 dans laquelle le pointeur 400 ne se déplace pas, c'est-à-dire que la crémaillère 40 ne tourne pas. Ici, chaque course morte 385 correspond à un parcours circulaire d'axe A. Ainsi, lorsque la deuxième roue dentée 38 est entraînée par l'actionneur 12, le pointeur 400 ne change pas de position. Les limites entre la course 386 pour le pivotement de l'insert 7 et les deux courses mortes 385a, 385b adjacentes sont représentées en trait en pointillé.

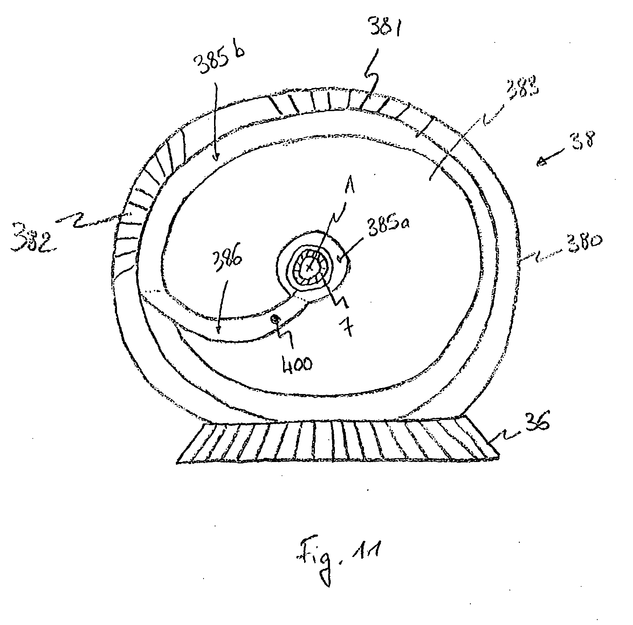

En figure 11, la deuxième roue dentée 38 est dans une position dans laquelle le pointeur 400 se situe dans la course 386 pour le pivotement de l'insert 7. Cette position est obtenue par rotation de la deuxième roue dentée 38 dans le sens anti-horaire en partant d'une position initiale représentée en figure 9. Le déplacement du pointeur 400 implique la rotation de la crémaillère 40 et de la roue crantée 76 et donc la rotation de l'insert 7 de manière à changer d'agent volatil à diffuser à l'intérieur de l'habitacle du véhicule, diffuser plus ou moins d'agent volatil (réglage de l'intensité de diffusion), ou assurer ou non la diffusion (cyclage).

Lorsque la rotation de la deuxième roue dentée 38 se poursuit dans le sens anti-horaire, le pointeur 400 parcoure toute la course 386, provoquant ainsi une rotation de 180 degrés de l'insert 7. Lorsque le pointeur 400 atteint la limite entre de la course 386 et la deuxième course morte 385b, il cesse de se déplacer.

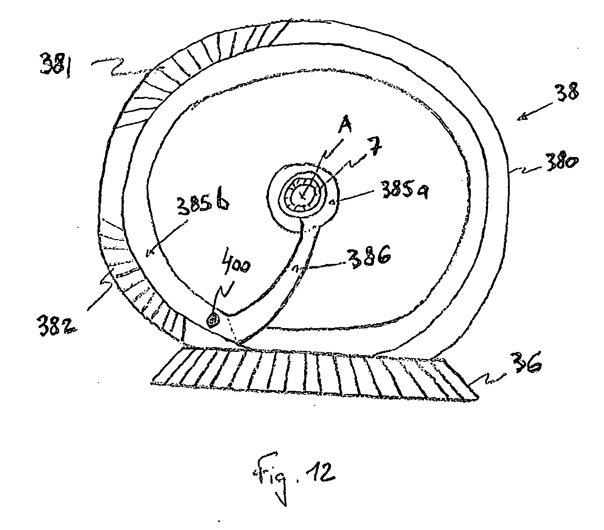

La figure 12 représente une position de la deuxième roue dentée 38 dans laquelle le pointeur 400 se situe dans la deuxième course morte 385b. Si l'actionneur continue d'entraîner dans le sens anti-horaire la deuxième roue dentée 38, le pointeur 400 ne se déplacera plus.

Le chemin de came 384 est positionnée sur la surface radiale de la deuxième roue dentée 38 de manière à engendrer un mouvement de rotation de l'insert 7 lorsque la première roue dentée 36 n'est pas entraînée en rotation par la deuxième roue 38. En effet, comme dans le premier mode de réalisation, l'extraction et l'insertion du tiroir 18 dans le boîtier 2 sont des étapes distinctes et non simultanées de l'étape de rotation de l'insert 7 pour un changement de diffusion de l'agent volatil.

Il est à noter que l'actionneur 12 entraîne en rotation la deuxième roue dentée 38 selon le sens horaire ou anti-horaire. En conséquence, un retour à la diffusion de l'agent volatil initial est possible par une simple rotation de l'actionneur 12 dans le sens horaire.

Ce mode de réalisation présente les avantages suivants. Les ressorts pour extraire le tiroir 18 sont supprimés ce qui assure une robustesse du système. En effet, un ressort peut perdre de sa force au fur et à mesure de son utilisation. Le dispositif de diffusion est plus facile à assembler, le montage des ressorts étant toujours délicats lorsqu'ils sont à l'état de compression. Un tel mode de réalisation est économique du fait qu'un seul moteur est requis pour toutes les opérations. En outre, selon ce mode de réalisation, les moyens de verrouillage et les moyens d'amortissement ne sont plus requis étant donné que le mouvement de translation du tiroir 18 est intégralement commandé par l'actionneur 12 et n'est plus amorcé par une action de pression axiale. Enfin, il n'y a plus d'effort manuel de la part de l'utilisateur sur le dispositif de diffusion lui-même et le dispositif de diffusion est plus sécurisé et plus simple puisque toutes les commandes se font au tableau de commande.

## Revendications

1. Dispositif (1) de diffusion d'au moins un agent volatil dans un véhicule automobile, comprenant un boîtier (2) comportant une entrée (3) d'air ambiant, une sortie (5) d'air traité et une chambre (6) de diffusion apte à recevoir un insert (7), au moins une cartouche (8, 9) d'agent volatil, ledit insert (7) comprenant un berceau (15) portant ladite cartouche (8, 9), **caractérisé en ce que** ledit dispositif comprend en outre un actionneur (12) entraînant l'insert (7) en rotation autour d'un axe de rotation A, ledit berceau (15) étant lié en rotation à l'insert (7) et monté libre en translation axiale sur l'insert (7), et **en ce que** ledit dispositif (1) comprend des moyens d'extraction dudit berceau (15) hors dudit boîtier (2) par translation parallèlement à l'axe A.

2. Dispositif de diffusion selon la revendication 1, dans lequel lesdits moyens d'extraction comprennent un tiroir (18) d'extraction logé dans la chambre (6) de diffusion et contenant ledit insert (7), et des moyens d'entraînement en translation axiale dudit tiroir (18) hors du boîtier (2), le tiroir (18) d'extraction étant apte à entraîner ledit berceau (15) hors du boîtier (2) sous l'action desdits moyens d'entraînement en translation axiale.

3. Dispositif de diffusion selon la revendication 2, dans lequel lesdits moyens d'entraînement sont bloqués pour toute position angulaire du berceau autour de l'axe A différente d'une position angulaire d'extraction donnée.

4. Dispositif de diffusion selon la revendication 3, dans lequel le berceau (15) est amené à ladite position angulaire d'extraction au moyen dudit actionneur (12).

5. Dispositif de diffusion selon l'une quelconque des revendications 2 à 4, dans lequel lesdits moyens d'entraînement en translation axiale sont activés par une action de pression axiale sur ledit tiroir (18) d'extraction.

6. Dispositif de diffusion selon la revendication 5, dans lequel lesdits moyens d'entraînement en translation axiale comprennent au moins un ressort apte à entraîner le tiroir (18) en translation axiale.

7. Dispositif de diffusion selon l'une des revendications 5 ou 6, comprenant des moyens de verrouillage (181, 21) du tiroir (18) d'extraction dans le boîtier (2), lesdits moyens de verrouillage étant aptes à être libérés sous ladite action de pression axiale.

8. Dispositif de diffusion selon la revendication 7, dans lequel lesdits moyens de verrouillage comprennent une came (181) ménagée sur le tiroir (18) et coopérant avec un ressort (21) de flexion disposé dans le boîtier (2).

9. Dispositif de diffusion selon l'une quelconque des revendications 3 à 8, dans lequel le tiroir (18) d'extraction et l'insert (7) comportent des moyens (185, 153, 154 ; 74) de butée destinés à bloquer la libération des moyens (181, 21) de verrouillage sous ladite action de pression axiale lorsque le berceau (15) est dans une position angulaire différente de ladite position angulaire d'extraction.

10. Dispositif de diffusion selon l'une quelconque des revendications 2 à 9, comprenant des moyens (183, 19) d'amortissement du mouvement de translation axiale du tiroir (18).

11. Dispositif de diffusion selon l'une quelconque des revendications 1 à 10, dans lequel ladite chambre (6) de diffusion présente un secteur (S) de plus grand diamètre destiné à limiter la compression radiale de moyens (81) d'étanchéité de la cartouche (8) en position d'extraction.

12. Dispositif selon l'une quelconque des revendications 2 à 11, comprenant un détecteur d'ouverture du tiroir apte à fournir un signal de mise hors fonctionnement dudit dispositif (1) lorsque le tiroir (18) est hors du boîtier (2).

13. Dispositif selon l'une quelconque des revendications 2 à 12, dans lequel le tiroir (18) est muni d'un bouchon (14) comportant des moyens (16) de distribution radiale de l'air traité.

14. Dispositif selon l'une quelconque des revendications 2 à 4, dans lequel le moyen d'entraînement est commandé par l'actionneur 12.

15. Dispositif de diffusion selon la revendication 14, dans lequel le moyen d'entraînement comprend au moins deux roues dentées 36, 38 et au moins deux bielles 30, 32.

## Claims

1. Device for (1) diffusing at least one volatile agent in a motor vehicle, comprising a case (2) comprising an ambient air inlet (3), a treated air outlet (5) and a diffusion chamber (6) able to receive an insert (7), at least one cartridge (8, 9) of volatile agent, said insert (7) comprising a cradle (15) carrying said cartridge (8, 9), **characterised in that** said device further comprises an actuator (12) driving the insert (7) in rotation around an axis of rotation A, said cradle (15) being linked in rotation to the insert (7) and being mounted freely in radial translation on the insert (7), and **in that** the said device (1) comprising means of extracting of said cradle (15) outside of said case (2) by translation in parallel to the axis A.

2. Device for diffusing according to claim 1, wherein said means of extracting include a draw (18) for extraction housed in the diffusion chamber (6) and containing said insert (7), and means of driving in axial translation of said drawer (18) outside of the case (2), the drawer (18) for extraction able to drive said cradle (15) outside of the case (2) under the action of said means of driving in axial translation.

3. Device for diffusing according to claim 2, wherein said means of driving are blocked for any angular position of the cradle around the axis A that is different from a given angular position of extraction.

4. Device for diffusing according to claim 3, wherein the cradle (15) is brought to said angular position of extraction by means of said actuator (12).

5. Device for diffusing according to any of claims 2 to 4, wherein said means of driving in axial translation are activated by an action of axial pressure on said drawer (18) for extraction.

6. Device for diffusing according to claim 5, wherein said means of driving in axial translation include at least one spring able to drive the drawer (18) in axial translation.

7. Device for diffusing according to one of claims 5 or 6, comprising means of locking (181, 21) of the drawer (18) for extraction in the case (2), said means of locking able to be released under said action of axial pressure.

8. Device for diffusing according to claim 7, wherein said means of locking include a came (181) arranged on the drawer (18) and cooperating with a spring subjected to bending stress (21) arranged in the case (2).

9. Device for diffusing according to any of claims 3 to 8, wherein the drawer (18) for extraction and the insert (7) comprise means (185, 153, 154; 74) of stopping intended to block the releasing of the means (181, 21) of locking under said action of axial pressure when the cradle (15) is in an angular position that is different from said angular extraction position.

10. Device for diffusing according to any of claims 2 to 9, comprising means (183, 19) of absorbing the movement of axial translation of the drawer (18).

11. Device for diffusing according to any of claims 1 to 10, wherein said diffusion chamber (6) has a sector (S) of greater diameter intended to limit the radial compression of the means (81) for sealing of the cartridge (8) in extraction position.

12. Device according to any of claims 2 to 11, comprising a detector of opening of the drawer able to provide a signal of taking out of operation of said device (1) when the drawer (18) is outside of the case (2).

13. Device according to any of claims 2 to 12, wherein the drawer (18) is provided with a plug (14) comprising means (16) of radial distribution of the treated air.

14. Device according to any of claims 2 to 4, wherein the means of driving is controlled by the actuator 12.

15. Device for diffusing according to claim 14, wherein the means of driving comprise at least two gears 36, 38 and at least two connecting rods 30, 32.

## Patentansprüche

1. Einrichtung (1) zur Diffusion von mindestens einem flüchtigen Mittel in einem Kraftfahrzeug, umfassend ein Gehäuse (2), das einen Raumlufteintritt (3), einen Austritt (5) der behandelten Luft und eine Diffusionskammer (6) umfasst, die imstande ist, einen Einsatz (7) aufzunehmen, mindestens eine Kartusche (8, 9) mit einem flüchtigen Mittel, wobei der Einsatz (7) einen Tragkorb (15) umfasst, der die Kartusche (8, 9) trägt, wobei der Tragkorb frei axial verschiebbar auf dem Einsatz (7) befestigt ist, wobei die Einrichtung (1) Mittel zur Entnahme des Tragkorbs (15) aus dem Gehäuse (2) durch Parallelverschiebung zu einer Achse A umfasst, **dadurch gekennzeichnet, dass** die Einrichtung ferner ein Stellglied (12) umfasst, das den Einsatz (7) rund um die Drehachse A in Drehung versetzt, und **dadurch**, dass der Tragkorb (15) mit dem Einsatz (7) drehverbunden ist.

2. Diffusionseinrichtung nach Anspruch 1, wobei die Entnahmemittel einen Entnahmeschieber (18), der in der Diffusionskammer (6) untergebracht ist und den Einsatz (7) enthält, und Mittel umfassen, um den Schieber (18) axial aus dem Gehäuse (2) zu schieben, wobei der Entnahmeschieber (18) imstande ist, den Tragkorb (15) unter Einwirkung der Mittel zur axialen Verschiebung aus dem Gehäuse (2) hinauszubewegen.

3. Diffusionseinrichtung nach Anspruch 2, wobei die Mittel zur Verschiebung bei jedweder Winkelposition des Tragkorbs rund um die Achse A blockiert werden, die sich von einer gegebenen Entnahmewinkelposition unterscheidet.

4. Diffusionseinrichtung nach Anspruch 3, wobei der Tragkorb (15) mithilfe des Stellglieds (12) in die Entnahmewinkelposition gebracht wird.

5. Diffusionseinrichtung nach einem der Ansprüche 2 bis 4, wobei die Mittel zur axialen Verschiebung durch eine axiale Druckwirkung auf den Entnahmeschieber (18) aktiviert werden.

6. Diffusionseinrichtung nach Anspruch 5, wobei die Mittel zur axialen Verschiebung mindestens eine Feder umfassen, die imstande ist, den Schieber (18) axial zu verschieben.

7. Diffusionseinrichtung nach einem der Ansprüche 5 oder 6, umfassend Mittel zur Verriegelung (181, 21) des Entnahmeschiebers (18) im Gehäuse (2), wobei die Verriegelungsmittel imstande sind, unter der axialen Druckwirkung freigegeben zu werden.

8. Diffusionseinrichtung nach Anspruch 7, wobei die Verriegelungsmittel einen Nocken (181) umfassen, der auf dem Schieber (18) angebracht ist und mit einer Biegefeder (21) zusammenwirkt, die auf dem Gehäuse (2) angeordnet ist.

9. Diffusionseinrichtung nach einem der Ansprüche 3 bis 8, wobei der Entnahmeschieber (18) und der Einsatz (7) Anschlagmittel (185, 153, 154; 74) umfassen, die zum Blockieren der Freigabe der Verriegelungsmittel (181, 21) unter der axialen Druckwirkung bestimmt sind, wenn sich der Tragkorb (15) in einer Winkelposition befindet, die sich von der Entnahmewinkelposition unterscheidet.

10. Diffusionseinrichtung nach einem der Ansprüche 2 bis 9, umfassend Mittel (183, 19) )zur Dämpfung der axialen Verschiebungsbewegung des Schiebers (18).

11. Diffusionseinrichtung nach einem der Ansprüche 1 bis 10, wobei die Diffusionskammer (6) einen Sektor (S) mit einem größeren Durchmesser aufweist, der dazu bestimmt ist, die radiale Kompression der Dichtungsmittel (81) der Kartusche (8) in Entnahmeposition zu begrenzen.

12. Einrichtung nach einem der Ansprüche 2 bis 11, umfassend einen Detektor der Öffnung des Schiebers, der imstande ist, ein Signal der Außerbetriebsetzung der Einrichtung (1) abzugeben, wenn sich der Schieber (18) außerhalb des Gehäuses (2) befindet.

13. Einrichtung nach einem der Ansprüche 2 bis 12, wobei der Schieber (18) mit einem Deckel (14) ausgestattet ist, der Mittel (16) zur radialen Verteilung der behandelten Luft umfasst.

14. Einrichtung nach einem der Ansprüche 2 bis 4, wobei das Mittel zur Verschiebung vom Stellglied (12) gesteuert wird.

15. Diffusionseinrichtung nach Anspruch 14, wobei das Mittel zur Verschiebung mindestens zwei Zahnräder (36, 38) und mindestens zwei Kolbenstangen (30, 32) umfasst.
